# EUROPEAN PATENT APPLICATION

(11) **EP 3 970 511 A1**
(43) Date of publication of application: **23.03.2022**
(21) Application number: 20805090.6
(22) Date of filing: 11.05.2020
(51) Int. Cl.: A23L 33/135, A61K 35/747, A61P 1/16, C12N 1/20, C12R 1/225

(54) **STRAIN SHOWING LIVER FUNCTION IMPROVING ACTIVITY, AND USE THEREOF**

(30) Priority: 14.05.2019 KR 20190056228; 08.04.2020 KR 20200042863
(71) Applicant: Korea Food Research Institute, Wanju-gun, Jeollabuk-do 55365 (KR)
(72) Inventor: PARK, Ho Young, Jeonju-si, Jeollabuk-do 54866 (KR); KIM, Yoon Sook, Seoul 04732 (KR); OH, Mi Jin, Wanju-gun, Jeollabuk-do 55365 (KR); LEE, Sang Hoon, Jeonju-si, Jeollabuk-do 54864 (KR); LEE, Hyun Hee, Seoul 02872 (KR); HA, Sang Keun, Daejeon 34069 (KR); HUR, Jin Young, Yongin-si, Gyeonggi-do 16836 (KR)
(74) Representative: Callaghan, Dayle Anne
(86) International application number: PCT/KR2020/006172
(87) International publication number: WO 2020/231127

(57) **Abstract**

The present invention relates to a strain having liver function improving activity, and a use thereof. Specifically, the present invention relates to a health food composition for improving liver function and a pharmaceutical composition for improving liver function, which contain, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof. The strain of the present invention shows excellent activity in inhibiting the activity of alanine aminotransferase (ALT) or aspartate aminotransferase (AST), inhibiting an increase in the concentration of total cholesterol or triglycerides in the liver, and inhibiting fat accumulation in the liver, thereby being effectively usable in the preparation of a food composition and a pharmaceutical composition for improving liver function.

## Description

### Technical Field

This application claims the priority benefit of Korean Patent Application No. 10-2019-0056228 filed on May 14, 2019 and Korean Patent Application No. 10-2020-0042863 filed on April 8, 2020, the contents of each of which are incorporated herein by reference in their entirety.

The present invention relates to a strain having liver function improving activity, and a use thereof. Specifically, the present invention relates to a health food composition and a pharmaceutical composition for improving liver function, using a Lactobacillus pentosus KF8 (accession number KCCM11997P) strain, a Bacillus subtilis KF11 (accession number KCCM11981P) strain, or a Lactococcus lactis KF140 (accession number KCCM11673P) strain.

### Background Art

The liver is known to act on blood storage and circulation, blood volume control and defense detoxification in the human body, and is closely related to mental activity. Our body is always exposed to pollutants and toxic substances caused by industrialization, so our liver is constantly suffering from detoxification.

In addition, the liver is an organ with a large buffering capacity, and the symptom does not appear well in the early stages of the disease and is found only when it is significantly worsened. Liver cirrhosis, liver cancer and the like are common last steps when various liver diseases develop chronically. The causes include alcohol, drugs, chemicals, viral hepatitis, biliary tract disease, metabolic diseases such as hematochromatosis, and autoimmune diseases, but the cause of the liver diseases is often unknown. Therefore, the liver is a very important organ for early health management.

According to the investigation of the recent health level of Koreans, the mortality rate from liver cancer was 23.4 per 100,000 people, the highest in the world, and the mortality rate from chronic liver disease was also the third with 28.8 per 100,000 people. In addition, the Korean National Statistical Office recently announced that liver disease was the highest cause of death in Korea with 56.1 cases per 100,000 people in their 40s, and liver disease is emerging as an important social problem. In recent years, the number of liver damage caused by mental stress is also increasing, so more research is being focused on improving liver function.

On the other hand, probiotics have been used in our diet for a long time, and in particular, beneficial effects such as intestinal regulating action, anticancer effect, and immune enhancing effect have been reported.

Accordingly, while studying the new physiological activity for the probiotics, in particular, a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, which were isolated and identified by the present inventors as a new strain through previous studies, the present inventors found that the above bacteria can effectively improve liver function. Based on the above, the present inventors completed the present invention.

### Prior Art Document

### Patent Document

(Patent Document 1) Korean Patent Publication No. 10-2018-0103772

### Detailed Description of the Invention

### Technical Problem

Therefore, an object of the present invention is to provide a health food composition for improving liver function, which contains, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof.

In addition, another object of the present invention is to provide a pharmaceutical composition for improving liver function, which contains, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof.

### Solution to Problem

In order to achieve the above object of the present invention, the present invention provides a health food composition for improving liver function, which contains, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof.

In one embodiment of the present invention, the composition may inhibit the activity of alanine aminotransferase (ALT) or aspartate aminotransferase (AST), inhibit an increase in the concentration of total cholesterol or triglycerides in the liver, or inhibit fat accumulation in the liver.

In one embodiment of the present invention, the health food composition may be any one formulation selected from powder, granules, pills, tablets, capsules, candies, syrups and beverages.

In addition, the present invention provides a pharmaceutical composition for improving liver function, which contains, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof.

In one embodiment of the present invention, the composition may inhibit the activity of alanine aminotransferase (ALT) or aspartate aminotransferase (AST), inhibit an increase in the concentration of total cholesterol or triglycerides in the liver, or inhibit fat accumulation in the liver.

In addition, the present invention provides a method for improving liver function, comprising administering or taking to a subject a composition containing, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof.

In addition, the present invention provides use of a composition containing, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof for improving liver function.

### Effects of the Invention

The present invention relates to a health food composition for improving liver function and a pharmaceutical composition for improving liver function, which contain, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof. The strain of the present invention shows excellent activity in inhibiting the activity of alanine aminotransferase (ALT) or aspartate aminotransferase (AST), inhibiting an increase in the concentration of total cholesterol or triglycerides in the liver, and inhibiting fat accumulation in the liver, thereby being effectively usable in the preparation of a food composition and a pharmaceutical composition for improving liver function.

### Brief Description of Drawings

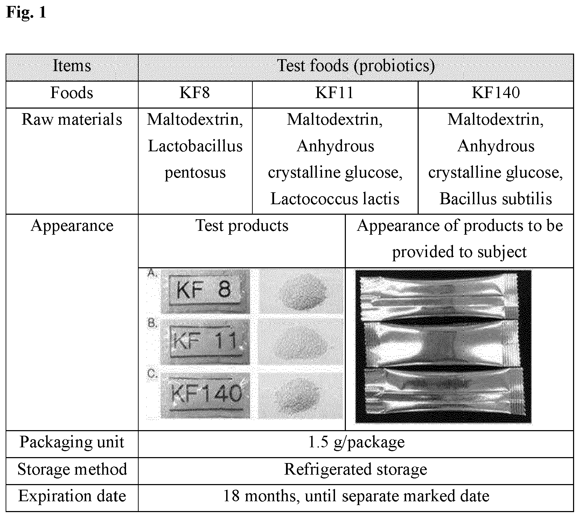

Fig. 1 illustrates a photograph showing the form of health food for improving liver function, which is prepared using a Lactobacillus pentosus KF8 (accession number KCCM11997P) strain, a Bacillus subtilis KF11 (accession number KCCM11981P) strain, and a Lactococcus lactis KF140 (accession number KCCM11673P) strain in one embodiment of the present invention.

### Best Mode for Carrying out the Invention

The present invention relates to a novel use of a strain having liver function improving activity, wherein the strain having liver function improving activity is a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, which were first isolated and identified by the inventors of the present invention and published previously.

The present inventors performed an experiment in order to determine whether the strain of the present invention can improve liver function and have liver protecting activity. As a result, it was found that as a result of administering the food form prepared by pulverizing the strain to an animal model for liver damage, the effect of protecting liver cells and inhibiting the activity of ALT and AST, which are indicators of liver damage, was excellent.

The ALT (aspartate aminotransferase), which is a representative liver damage indicator, is an enzyme present in the liver, kidney, heart, muscle, and the like, and is present in the largest amount in the liver. The ALT is used as an indicator of liver disease because it reacts sensitively when liver cells are damaged and the level is increased. When the liver is damaged for a variety of reasons, it is mainly released into the blood and the blood level is elevated.

In addition, AST (alanine aminotransferase), which is another liver damage indicator, is also released into the blood during liver damage, and the blood level is elevated compared to the normal condition. Therefore, the level of liver damage can be confirmed by measuring the content of these indicators.

On the other hand, as a result of administering a Lactobacillus pentosus KF8 (accession number KCCM11997P) strain, a Bacillus subtilis KF11 (accession number KCCM11981P) strain, or a Lactococcus lactis KF140 (accession number KCCM11673P) strain, which is the strain of the present invention, to a mouse group in which liver damage is induced, it was found that it had an effect of reducing the increased ALT and AST levels in the blood by two times or more.

In addition, in one embodiment of the present invention, it was analyzed whether the content of total cholesterol and triglycerides increased in the liver according to the onset of fatty liver could be reduced when the strain of the present invention was administered. As a result of administering the strain of the present invention to a fatty liver animal model, it was found that the contents of total cholesterol and triglycerides in the liver were effectively inhibited, and it was found that fat accumulation in the liver was inhibited.

It is known that fatty liver is caused by inhibition of the formation and release of lipoproteins in the liver or by enhancement of triglyceride synthesis. Ethionine inhibits the synthesis of RNA and protein and reduces the production of ATP in the liver, thereby inhibiting the excretion of triglycerides into the plasma, and also inhibits the synthesis of lipoproteins, thereby inhibiting the release of lipids from the liver. Since the release is inhibited, triglycerides are accumulated in the liver tissue when ethionine is administered, and the amount of triglycerides and total cholesterol in the blood is rapidly lowered, causing microvesicular steatosis. Therefore, in the ethionine-induced fatty liver model, the triglyceride content in the liver tissue and the total cholesterol content in the serum are used as indicators of the model.

In this regard, it can be seen that the strain of the present invention is useful for improving and treating fatty liver because it has been confirmed that it has an effect of reducing triglycerides and total cholesterol in liver tissue.

Furthermore, in another embodiment of the present invention, as a result of performing a clinical trial on volunteers conducted in accordance with the regulations of the Institutional Review Board (IRB), it was found that in the case of the group who ingested the health food prepared using the strain of the present invention, the activity of ALT and AST in the blood was inhibited compared to the group who did not ingest the same, thereby having an effect of improving liver function, and it was found that the contents of total cholesterol and LDL cholesterol were also reduced by ingestion of the strain of the present invention.

Therefore, through these results, the present inventors found that a Lactobacillus pentosus KF8 (accession number KCCM11997P) strain, a Bacillus subtilis KF11 (accession number KCCM11981P) strain, or a Lactococcus lactis KF140 (accession number KCCM11673P) strain, which was first isolated and identified by the present inventors, can be used for the purpose of improving liver function.

Therefore, the present invention may provide a health food composition for improving liver function, which contains, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof.

The number of the above strains that may be contained in the health food composition for improving liver function of the present invention may be 1.0×10⁵ CFU/g to 2.0×10¹⁵ CFU/g, and the health food composition of the present invention may include one type of strain among the above strains, or may include one or more types of mixed strains, and most preferably, it may include mixed strains in which all three types of strains are mixed.

The effect of improving liver function of the Lactobacillus pentosus KF8 (accession number KCCM11997P) strain, the Bacillus subtilis KF11 (accession number KCCM11981P) strain, or the Lactococcus lactis KF140 (accession number KCCM11673P) strain investigated in the experiment of the present invention, i.e., the effects of inhibiting the activity of ALT and AST in the blood, inhibiting total cholesterol or triglycerides in the liver tissue, and inhibiting fat accumulation in the liver, can also be induced by the treatment with the single strain alone, and when two or more strains are mixed, a better effect can be exhibited, and when all three strains are mixed, the best effect can be exhibited.

The food composition of the present invention includes all forms of a functional food, a nutritional supplement, a health food, a food additive, and the like. The food composition of the above type may be manufactured in various forms according to the conventional methods known in the art. For example, as a health food, at least one of the group of the strains of the present invention itself, a lysate thereof, and a culture product of the above strain may be drunken in the form of tea, juice, and drink, and may be ingested by granulation, encapsulation, and pulverization. In addition, at least one of the group of the strains of the present invention, a lysate thereof, and a culture product thereof may be manufactured in the form of a composition by mixing with a known substance or an active ingredient known to have an effect of improving liver function. In addition, a functional food may be manufactured by adding at least one of the group of the strains of the present invention, a lysate thereof, and a culture product thereof to beverages (including alcoholic beverages), fruits and their processed foods (for example, a canned fruit, a bottled fruit, jam, marmalade, etc.), fish, meat, and its processed food (for example, ham, sausage corned beef, etc.), breads and noodles (for example, udon, buckwheat noodle, ramen, spaghetti, macaroni, etc.), fruit juice, various drinks, cookies, taffy, dairy products (for example, butter, cheese, etc.), edible vegetable oil and fat, margarine, vegetable protein, retort food, frozen food, various seasonings (for example, bean paste, soy sauce, sauce, etc.), and the like.

The preferable content of the strain of the present invention, a lysate thereof, and a culture product thereof, etc. in the food composition of the present invention is preferably, but not limited to, 0.01 to 50 wt% in a finally manufactured food. In addition, in order to use in the form of a food additive, the strain of the present invention, a lysate thereof or a culture product thereof as an active ingredient may be manufactured in the form of powder or concentrate.

In addition, the present invention may provide a pharmaceutical composition for improving liver function, which contains, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof.

The pharmaceutical composition may contain a pharmaceutically acceptable salt alone or may further contain one or more pharmaceutically acceptable carriers, excipients, or diluents. The pharmaceutically acceptable carrier may further include, for example, a carrier for oral administration or a carrier for parenteral administration. Carriers for oral administration may include lactose, starch, cellulose derivatives, magnesium stearate, stearic acid, and the like. In addition, carriers for parenteral administration may include water, a suitable oil, saline, aqueous glucose, glycol, and the like, and may further include a stabilizer and a preservative. Suitable stabilizers include antioxidants such as sodium hydrogen sulfite, sodium sulfite or ascorbic acid. Suitable preservatives include benzalkonium chloride, methyl- or propyl-paraben and chlorobutanol. As other pharmaceutically acceptable carriers, reference may be made to those described in the literature (Remington's Pharmaceutical Sciences, 19th ed., Mack Publishing Company, Easton, PA, 1995).

The pharmaceutical composition of the present invention may be administered by any method to a mammal including a human. For example, it may be administered orally or parenterally. A parenteral administration method includes intravenous, intramuscular, intraarterial, intramedullary, intrathecal, intracardiac, transdermal, subcutaneous, intraperitoneal, intranasal, intestinal, topical, sublingual, or intracolonic administration, but is not limited thereto. Preferably, the pharmaceutical composition of the present invention may be administered orally or transdermally.

The pharmaceutical composition of the present invention may be formulated in a formulation for oral administration or a formulation for parenteral administration by the routes of administration as described above.

In the case of a formulation for oral administration, the composition of the present invention may be formulated using the methods known in the art into powder, granules, tablets, pills, dragees, capsules, liquids, gels, syrups, slurries, suspensions, and the like. For example, a formulation for oral administration may be obtained as a tablet or a dragee by blending an active ingredient with a solid excipient, milling them, adding a suitable adjuvant, and then, processing into a granule mixture. Examples of suitable excipients may include sugars, such as lactose, dextrose, sucrose, sorbitol, mannitol, xylitol, erythritol, maltitol, and the like, starches, such as corn starch, wheat starch, rice starch, potato starch, and the like, celluloses, such as cellulose, methyl cellulose, sodium carboxymethyl cellulose, hydroxypropyl methylcellulose, and the like, and fillers, such as gelatin, polyvinylpyrrolidone, etc. In addition, in some cases, cross-linked polyvinylpyrrolidone, agar, alginic acid, or sodium alginate, etc. may be added as a disintegrating agent. Furthermore, the pharmaceutical composition of the present invention may further comprise anticoagulants, lubricants, wetting agents, flavoring agents, emulsifiers, preservatives, and the like.

The formulation for parenteral administration may be formulated in the form of injections, creams, lotions, external ointments, oils, moisturizers, gels, aerosols, and nasal inhalers using the methods known in the art. These formulations are described in the literature (Remington's Pharmaceutical Science, 15th Edition, 1975. Mack Publishing Company, Easton, Pennsylvania 18042, Chapter 87: Blaug, Seymour), which is a formulary generally known in all pharmaceuticals and chemistry.

The total effective amount of the strain of the present invention itself, a lysate thereof, and a culture product of the above strain, etc., may be administered to a patient as a single dose, and may be administered by fractionated treatment protocol, that is a long-term administration of multiple doses. The content of the active ingredient in the pharmaceutical composition of the present invention may vary depending on the severity of the disease. The preferable total dose of the strain of the present invention, a lysate thereof, and a culture product thereof may be preferably about 0.01 ug to 1,000 mg, most preferably 0.1 ug to 100 mg, per 1 kg of patient's body weight per day. However, the dose of the strain of the present invention itself, a lysate thereof, and a culture product of the above strain, etc., is determined upon consideration of routes of administration of the pharmaceutical composition and the number of times being treated, as well as various factors, such as patient's age, body weight, health condition, sex, severity of diseases, diet, excretion rate, and the like, for determining the effective dose for the patient. Thus, upon consideration of such aspects, a person having ordinary skill in the art would be able to determine an appropriate effective dose according to the certain use of the strain of the present invention, a lysate thereof, and a culture product thereof as an immune function enhancer. The pharmaceutical composition according to the present invention is not particularly limited to the formulations, routes of administration, and administration methods as long as the effect of the present invention shows.

Furthermore, in the present invention, improvement of liver function refers to treatment, alleviation, and prevention of symptoms caused by liver damage, and means to treat, alleviate or prevent liver function, liver condition, liver disease, and liver disorder.

In the present invention, liver disease that can be caused by liver damage may include autoimmune liver disease, drug-induced liver disease, alcoholic liver disease, infectious liver disease, congenital metabolic liver disease, acute liver disease, hepatitis, bile stasis, fatty liver and chronic liver disease, but is not limited thereto.

Hereinafter, the present invention will be described in more detail through the examples. These examples are for illustrating the present invention in more detail, and the scope of the present invention is not limited to these examples.

### <Preparation Example>

### <1> Preparation of test food containing the strain of the present invention

The present inventors requested a strain depositary authority to assign an accession number for three strains previously isolated and identified as new novel strains, and requested Mediogen Co., Ltd. to prepare the test food using a total of three strains deposited (Lactobacillus pentosus KF8, Bacillus subtilis KF11, and Lactococcus lactis KF140).

A total of three strains used in the present invention are as follows.
- Lactobacillus pentosus KF8: a strain that was deposited on March 24, 2017 at the Korean Culture Center of Microorganisms (KCCM), which is an international microorganism depositary authority, and assigned with an accession number KCCM 11997P was used.
- Bacillus subtilis KF11: a strain that was deposited on February 24, 2017 at the Korean Culture Center of Microorganisms (KCCM), which is an international microorganism depositary authority, and assigned with an accession number KCCM 11981P was used.
- Lactococcus lactis KF140: a strain that was deposited at the Korean Culture Center of Microorganisms (KCCM), which is an international microorganism depositary authority, and assigned with an accession number KCCM 11673P was used.

Preparation of test foods using these strains was requested to Mediogen Co., Ltd. and prepared according to the conventional method for producing strain-type products. The main raw materials, including maltodextrin, anhydrous crystalline glucose, and each of the three strains of the present invention (2.0 × 10⁹ CFU/g), were packaged in a unit of 1.5 g per package and kept refrigerated until used for the test.

At this time, each of the strains used for the preparation of the test food was cultured in a culture medium to obtain a strain, and then it was lyophilized and pulverized to obtain the strain in the form of a powder and use it.

A photograph of the test food prepared for use in the present invention is shown in Fig. 1.

### <2> Test subject

In order to confirm the effect of improving liver function on the three strains of the present invention, the test subjects were selected as follows.

Subjects were selected as those who met the criteria below, and they volunteered to recruit subjects for human application tests, and the following experiment was conducted for only those who passed the selection criteria. A screening test was conducted for a total of 44 adults, aged 25 to 45 years of age, residing in Daegu and Gyeongbuk area, and 36 people who met the selection criteria and did not meet the exclusion criteria were selected as subjects.

Subject selection criteria were limited to those who were able to follow-up during the trial period, and those who voluntarily signed the consent form after fully explaining the purpose and content of the study.

On the other hand, those who met the following conditions were excluded.
- Those who have experience of hypersensitivity reaction when ingesting strain products
- Those who have ingested prescription drugs, over-the-counter drugs, health functional foods, or probiotics within the past month
- Those who have ingested antibiotics within 1 month of participating in the test
- Those who have undergone endoscopic examination of stomach or large intestine within 1 month of participating in the test
- Those who have a BMI of less than 20 or greater than 35
- Those who have high blood pressure (systolic blood pressure of 160 mmHg or

diastolic blood pressure of 100 mmHg or higher)
- Those who have a fasting blood glucose concentration of greater than 110
- Those who have ALT or AST exceeding 2 times the upper limit of normal
- Women who are pregnant or lactating
- Women of childbearing age who do not consent to contraception using medically proven methods (e.g., condoms, loops, femidoms, etc.) during the test period
- Those who need continuous treatment for anorexia, depression, bipolar disorder, psychiatric disease, etc.
- Those who have systemic diseases such as immune-related diseases, severe liver or kidney failure, malignant tumors, lung diseases, collagenosis, multiple sclerosis, allergic skin diseases, and other autoimmune diseases
- Those who have a history of gastrointestinal disease or gastrointestinal surgery (excluding simple appendectomy or hernia surgery) that may affect the absorption of the test food
- Those who have participated in other human application tests or clinical trials within 3 months of participating in the test and have ingested the test product (excluding cosmetics human application tests)
- Those who are judged to be difficult to conduct the test in the judgment of the researcher other than the above

In addition, subjects who met the following conditions were excluded from this test due to the questionnaire on the day of hospitalization.
- Those who have ingested alcohol in excess of 50 g (one or more bottle of soju or two or more bottles of beer) per serving within 2 weeks before hospitalization
- Those who have consumed more than 1 glass of soju or 1 glass of beer per day within 1 week before hospitalization
- Those who have ingested dairy products, fast foods such as hamburgers and pizzas, instant foods, fried foods, grilled foods, etc. within 1 week before hospitalization
- Those who have ingested antibiotics after the date of previous visit
- Those who have undergone endoscopic examination of stomach or large intestine after the date of previous visit
- Those who have taken prescription drugs, over-the-counter drugs, or health functional foods after the date of the previous visit

Subjects selected under these conditions were set to be 10 people per experimental group as the minimum number of subjects that could be analyzed for effectiveness.

The clinical trials of the selected subjects were conducted according to the regulations of the Institutional Review Board (IRB).

### <Example 1>

### Analysis of the effect of improving liver function according to ingestion of the strain of the present invention

Changes in AST, ALT, total cholesterol, triglyceride, LDL cholesterol, and HDL cholesterol in the blood were measured using blood collected before and after ingestion of each strain for 26 days for the test subjects.

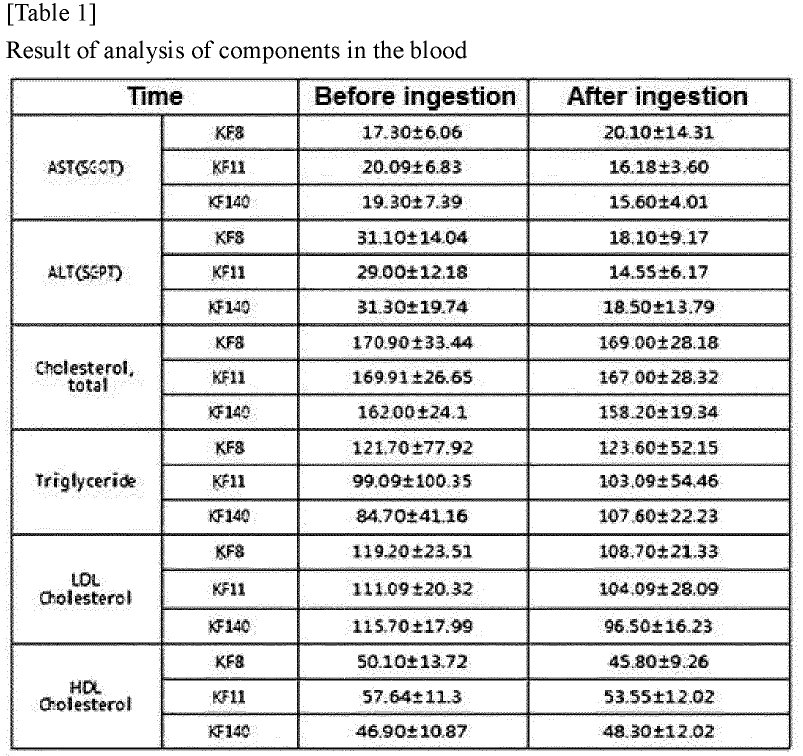

As a result of the analysis, as shown in Table 1 above, it was found that in the group that ingested each of the strains of the present invention, Lactobacillus pentosus KF8, Bacillus subtilis KF11, and Lactococcus lactis KF140, the levels of alanine aminotransferase (ALT) and aspartate aminotransferase (AST), indicators of liver function were effectively inhibited compared to those before ingestion of the strain.

Therefore, through these results, the present inventors found that the strains of Lactobacillus pentosus KF8, Bacillus subtilis KF11, and Lactococcus lactis KF140 can be used for the purpose of improving liver function and preventing and treating liver disease.

### <Example 2>

### Analysis of the effect of improving liver function according to ingestion of the strain of the present invention in a liver damage-induced animal model

As experimental animals, male SD (Sprague-Dawley) rats (SLC, Japan) having a body weight of 200 g were used, and 5 animals were distributed to each experimental group and used for the experiment. Each experimental group is as follows.

**[Table 2]**

| | Content of administration |
|---|---|
| Normal group | ingestion of normal diet |
| Control group | normal diet + intraperitoneal administration of carbon tetrachloride |
| Experimental group 1 | normal diet + intraperitoneal administration of carbon tetrachloride + administration of Lactobacillus pentosus KF8 |
| Experimental group 2 | normal diet + intraperitoneal administration of carbon tetrachloride + administration of Bacillus subtilis KF11 |
| Experimental group 3 | normal diet + intraperitoneal administration of carbon tetrachloride + administration of Lactococcus lactis KF140 |

At this time, the carbon tetrachloride was intraperitoneally administered twice a week for 4 weeks at a dose of 0.75 ml/kg to the rat as a 50% solution mixed with corn oil. 50 mg/kg of each of the functional powder food containing the strain of the present invention prepared in Preparation Example was suspended in a 1% CMC solution and orally administered once a day, 6 times a week to the experimental groups. Thereafter, the administration was continued for 4 weeks, and then the animals were sacrificed to obtain the serum. In order to examine the therapeutic effect of the strain of the present invention on the liver damage induced by carbon tetrachloride, the activity of ALT and AST was measured.

**[Table 3]**

| Result of measurement of ALT and AST concentration | | |
|---|---|---|
| | ALT (U/T) | AST (U/T) |
| Normal group | 70±10 | 85±22 |
| Control group | 182±12 | 200±42 |
| Experimental group 1 (KF8) | 92±21 | 100±13 |
| Experimental group 2 (KF11) | 87±17 | 98±21 |
| Experimental group 3 (KF140) | 89±22 | 96±35 |

As a result of the analysis, as shown in Table 3 above, it was found that in the mouse group that ingested the strain of the present invention, the elevated ALT and AST in the serum due to carbon tetrachloride were effectively inhibited.

These results indicate that the Lactobacillus pentosus KF8, Bacillus subtilis KF11 and Lactococcus lactis KF140 strains of the present invention have excellent hepatoprotective effects against hepatotoxicity and liver function effects.

### <Example 3>

### Analysis of the improvement effect according to ingestion of the strain of the present invention in a fatty liver animal model

As experimental animals, male C57BL/6 mice (Central, Korea) having a body weight of 20 g were used, and 9 animals were distributed to each experimental group and used for the experiment. Each experimental group is as follows. A high fat diet (40% fat) was provided in pellet form as a fatty liver inducing substance, and the CML reaction product was orally administered daily at a dose of 10 mg/kg. Thereafter, the strain of the present invention was administered to each mouse, and then the degree of improvement of fatty liver was analyzed.

**[Table 4]**

| | Content of administration |
|---|---|
| Normal group | ingestion of normal diet |
| Control group | high fat diet + CML diet |
| Experimental group 1 | high fat diet + CML diet + administration of Lactobacillus pentosus KF8 |
| Experimental group 2 | high fat diet + CML diet + administration of Bacillus subtilis KF11 |
| Experimental group 3 | high fat diet + CML diet + administration of Lactococcus lactis KF140 |

At this time, 1×10⁷ CFU of each of the strains of the present invention was suspended in a 0.9% saline solution and orally administered once a day, 6 times a week to the experimental groups. Thereafter, the administration was continued for 4 weeks, and then the animals were sacrificed to collect the blood, and then the contents of liver function-related enzymes and triglycerides were measured.

**[Table 5]**

| Result of measurement of concentration of liver function-related enzymes and triglycerides | | | |
|---|---|---|---|
| | AST (U/L) | ALT (U/L) | Triglyceride (mg/g) |
| Normal group | 44.5±1.7 | 19.7±1.1 | 62.3±7.2 |
| Control group | 64.2±1.2 | 31.2±2.0 | 85.5±7.2 |
| Experimental group 1 (KF8) | 48.4±2.6 | 24.4±2.8 | 68.4±5.0 |
| Experimental group 2 (KF11) | 48.6±3.4 | 27.5±1.6 | 66.3±7.4 |
| Experimental group 3 (KF140) | 43.1±2.5 | 24.3±1.7 | 59.5±6.8 |

As a result of the analysis, as shown in Table 5 above, it was found that in the mouse group that ingested the strain of the present invention, the levels of AST and ALT increased by liver damage were significantly inhibited, and an effect of preventing an increase in the content of triglycerides in the same manner was confirmed.

Through these results, the present inventors found that the Lactobacillus pentosus KF8, Bacillus subtilis KF11 or Lactococcus lactis KF140 strain of the present invention can be effective in preventing liver damage caused by glycotoxin ingestion, thereby improving and treating liver diseases such as fatty liver.

As described above, the present invention has been described mainly based upon the preferable examples. A person having ordinary skill in the art to which the present invention belongs would be able to understand that the present invention may be implemented in a modified form within the scope which does not deviate from the essential characteristics of the present invention. Therefore, the examples disclosed above should be considered from an explanatory point of view, not a limited point of view. The scope of the present invention is defined by the claims, not the foregoing description, and all of the differences within the scope equivalent thereto should be interpreted to be included in the scope of the present invention.

### [Accession number]

Depositary authority name: Korean Culture Center of Microorganisms (foreign country) Accession number: KCCM11997P

Deposit date: March 24, 2017

Depositary authority name: Korean Culture Center of Microorganisms (foreign country) Accession number: KCCM11981P

Deposit date: February 24, 2017

Depositary authority name: Korean Culture Center of Microorganisms (foreign country) Accession number: KCCM11673P

Deposit date: March 6, 2015

## Claims

1. A health food composition for improving liver function, which contains, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof.

2. The health food composition for improving liver function according to claim 1, **characterized in that** the composition inhibits the activity of alanine aminotransferase (ALT) or aspartate aminotransferase (AST), inhibits an increase in the concentration of total cholesterol or triglycerides in the liver, or inhibits fat accumulation in the liver.

3. The health food composition for improving liver function according to claim 1, **characterized in that** the health food composition is any one formulation selected from powder, granules, pills, tablets, capsules, candies, syrups and beverages.

4. A pharmaceutical composition for improving liver function, which contains, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof.

5. The pharmaceutical composition for improving liver function according to claim 4, **characterized in that** the composition inhibits the activity of alanine aminotransferase (ALT) or aspartate aminotransferase (AST), inhibits an increase in the concentration of total cholesterol or triglycerides in the liver, or inhibits fat accumulation in the liver.

6. A method for improving liver function, comprising administering or taking to a subject a composition containing, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof.

7. Use of a composition containing, as an active ingredient, one or more strains selected from the group consisting of a Lactobacillus pentosus KF8 (accession number KCCM11997P) strain, a Bacillus subtilis KF11 (accession number KCCM11981P) strain, and a Lactococcus lactis KF140 (accession number KCCM11673P) strain, a lysate thereof or a culture product thereof for improving liver function.
